# EUROPEAN PATENT SPECIFICATION

(11) **EP 0 873 416 B1**
(45) Date of publication and mention of the grant of the patent: **24.05.2000**
(21) Application number: 96941766.6
(22) Date of filing: 11.12.1996
(51) Int. Cl.: C12Q 1/00, C12Q 1/34, C12Q 1/37

(54) **METHOD FOR MONITORING ENZYMES**
VERFAHREN ZUR ÜBERWACHUNG VON ENZYMEN
PROCEDE DE SURVEILLANCE DES ENZYMES

(30) Priority: 12.12.1995 GB 9525403
(43) Date of publication of application: 28.10.1998
(73) Proprietor: University of Sunderland, Sunderland, Tyne & Wear SR2 7EE (GB)
(72) Inventor: TANG, Lian, Xiang, Sunderland Tyne & Wear SR1 3SW (GB); ROWELL, Frederick, John, Low Pittington Durham DH6 1BG (GB)
(74) Representative: Stuttard, Garry Philip
(86) International application number: GB9603052
(87) International publication number: WO9721831

(56) References cited:
- ANAL. PROC. , vol. 32, no. 12, July 1995, pages 255-256, XP000670989 L. X. TANG ET AL.: "Flow injection fluorescence measurement of collagenase using a mini-bioreactor with immobilized collagen labelled with Lucifer Yellow."
- ANALYST, vol. 120, July 1995, pages 1949-1952, XP000670925 L. X. TANG ET AL.: "Rapid sensitive assay for some protease enzymes using a fluorosubstrate-immobilized bioreactor."
- ANAL. PROC., vol. 32, no. 12, 1995, pages 519-520, XP000671430 L. X. TANG ET AL.: "Ultra-rapid fluorescence assay for cellulase using a substrate-immobilized mini-bioreactor."
- ANN.OCCUP. HYG., vol. 40, no. 4, 1996, pages 381-389, XP000670919 L. X. TANG ET AL.: "Development of near real-time monitoring systems for some serine proteases enzymes in the industrial atmosphere."

## Description

The present invention is concerned with monitoring for the presence and/or determining the concentration of enzymes and in particular with a method which makes possible such monitoring on a continuous basis.

Enzymes of various types are commonly used both in scientific research and in manufacturing industry, in particular the detergent and food industries. However it is important to monitor the exposure of workers to enzymes because some enzymes can have deleterious health effects, for example leading to respiratory sensitisation. Ideally monitoring for enzymes should be carried out either at regular intervals to ensure that exposure limits are not exceeded or more preferably continuously to detect concentrations above such limits and thereby make possible immediate corrective action.

Available methods for monitoring enzymes involve sequences of operations which typically include collecting an air sample, extracting solid material from the air, eluting the enzymes into solution, initiating the enzyme reaction, producing a derivative of the reaction product and measuring the quantity of the derivative by, for example, a spectrophotometric method. Such sequences of steps are inevitably time-consuming, are necessarily carried out in a laboratory away from the monitoring site, and therefore are generally unsuitable for continuous or frequent on-site monitoring.

It is an object of the present invention to provide an improved method of monitoring enzymes, which is suitable for use on a continuous basis or for monitoring at frequent short intervals.

The method according to the present invention for monitoring for the presence of an enzyme selected from the group consisting of cellulase enzymes, lipase enzymes and amylase enzymes comprises producing a substrate for a said enzyme, which substrate has been modified by labelling it with a fluorophore, exposing the labelled substrate to the enzyme, and monitoring the fluorescent-labelled reaction products thereby produced. The fluorescence response, for example as determined by means of a fluorimeter, has proved to be a reliable and effective indication of the presence and, subject to calibration, the concentration of the enzyme detected.

Enzymes, to the monitoring of which the method of the present invention may be applied, are enzymes of the cellulase, lipase, and amylase types.

The substrate used in the method is selected from among those with which the enzyme to be monitored reacts. Thus, by way of example, cellulase have been successfully monitored using labelled cellulose.

The fluorophore used in the method of the invention to label the substrate is selected to be readily and consistently detectable by normal fluorometric methods. Preferably the selected fluorophore is not influenced by variations in ambient conditions such as pH level. The preferred fluorophore is lucifer yellow. Less preferred, since the fluorescence signal arising is markedly influenced by pH levels, are fluorescein and its derivatives, for example fluorescein isothiocyanate (FITC) .

The method can be put into practice in various ways but it is much preferred to practice it on a continuous basis by conveying the air which contains the enzyme continuously into a collector containing a solution which is then passed over a fixed bed of the labelled substrate in a reactor, in particular a small reaction vessel which may be carried to, or installed at, the place to be monitored. The labelled substrate is in many cases advantageously carried upon a suitable support, for example glass beads or magnetisable particles of cellulose containing iron oxide. In the case of cellulose however, in view of its insolubility in water, the substrate may be used as solid particles without such support.

In one alternative way of practising the method according to the present invention, the labelled substrate is supported upon an optical fibre and the fluorescence signal along the interior of the fibre is monitored continuously. The signal will initially have a relatively higher value and, as the labelled reaction products break away from the fibre in response to the presence of enzyme, the signal will drop accordingly.

The reproducibility and sensitivity of the results obtained by the method of the invention is improved by choosing a solution of a buffer of the correct pH for passing through the reactor. For example, in the case of cellulose-based reactions, the use of acetate buffer is to be recommended, to maintain the pH value at a level corresponding to maximal enzyme activity. The reproducibility of the fluorescence signals is further enhanced by the inclusion of a small quantity of a detergent in the liquid.

The method according to the invention is illustrated, by way of example only, by means of the following Example.

### Example

Cellulose particles activated and then labelled with the fluorophore lucifer yellow were packed into a mini-column and this bioreactor was fed, at a flow rate of 2 ml/min, with samples containing 0.5 to 10 units per millilitre of cellulase activity, buffered with 0.05 mol/l acetate (pH 5.0), which also contains 0.1% (v/v) of the detergent Tween 20 (sorbitan monolaurate polyoxyalkylene). Within a response time of 30 seconds, a fluorescence peak was observed, the height of which was found to correspond to the enzyme activity.

## Claims

1. A method for monitoring for the presence of an enzyme selected from the group consisting of cellulase enzymes, lipase enzymes and amylase enzymes, characterised in that it comprises producing a substrate for a said enzyme, which substrate has been modified by labelling it with a fluorophore, exposing the labelled substrate to the enzyme, and monitoring the fluorescent-labelled reaction products thereby produced.

2. A method according to Claim 1, characterised in that the fluorescent-labelled reaction products are monitored by means of a fluorimeter.

3. A method according to either of the preceding claims, characterised in that the enzyme is a cellulase enzyme and the substrate is cellulose.

4. A method according to any of the preceding claims, characterised in that the fluorophore is lucifer yellow.

5. A method according to any of Claims 1 to 3, characterised in that the fluorophore is fluorescein or a fluorescein derivative.

6. A method according to any of the preceding claims, characterised in that it is carried out on a continuous basis by conveying air containing the enzyme continuously into a solution and then passing the resulting enzyme solution ever a fixed bed of the labelled substrate.

7. A method according to any of the preceding claims, characterised in that the labelled substrate is carried on a support.

8. A method according to Claim 7, characterised in that the support comprises glass beads or comprises cellulose particles containing iron oxide.

9. A method according to Claim 7, characterised in that the support is an optical fibre.

10. A method according to any of Claims 6 to 9, characterised in that the solution contains a buffer.

11. A method according to Claim 10, characterised in that the buffer is an acetate buffer or tris-(hydroxymethyl)-aminomethane.

## Patentansprüche

1. Jede Methode zur Überwachung der Präsenz eines Enzyms aus der Gruppe der Cellulasenenzyme, der Lipasenenzyme und der Amylasenenzyme, charakterisiert dadurch daß sie die Produktion eines Substrates für eines der oben genannten Enzyme beinhaltet, welches durch eine Markierung mit einem Fluorophor modifiziert worden ist, das so markierte Substrat mit dem Enzym in Verbindung bringt und die entstehenden Reaktionsprodukte mit Hilfe der Fluoreszens überwacht.

2. Eine Methode nach Anspruch 1, gekennzeichnet dadurch, daß die durch Fluoreszens gekennzeichneten Reaktionsprodukte mit Hilfe eines Fluorimeters oder Fluoreszensspektrometers überwacht werden.

3. Eine Methode nach einem der vorherigen Ansprüche, gekennzeichnet dadurch, daß das Enzym das Cellulaseenzym ist und das Substrat Cellulose.

4. Eine Methode nach jedem der vorherigen Ansprüche, gekennzeichnet dadurch, daß das Fluorophor Luciferyellow ist.

5. Eine Methode nach jedem der vorherigen Ansprüche 1-3, gekennzeichnet dadurch, daß das Fluorophor Fluorescein oder ein Fluoresceinderivat ist.

6. Eine Methode nach jedem der vorherigen Ansprüche, gekennzeichnet dadurch, daß sie kontinuierlich durchgeführt wird und die das Enzym enthaltene Luft kontinuierlich in eine Lösung transportiert und dann die entstehende Enzymlösung über ein fixiertes Bett des markierten Substrates führt.

7. Eine Methode nach jedem der vorherigen Ansprüche, gekennzeichnet dadurch, daß das markierte Substrat auf einem Träger aufgebracht wird.

8. Eine Methode nach Anspruch 7, gekennzeichnet dadurch, daß der Träger aus Glasperlen oder aus Eisenoxid enthaltenen Cellulosepartikeln besteht.

9. Eine Methode nach Anspruch 7, gekennzeichnet dadurch, daß der Träger eine optische Faser ist.

10. Eine Methode nach jedem der vorherigen Ansprüche 6 bis 9, gekennzeichnet dadurch, daß die Lösung einen Puffer enthält.

11. Eine Methode nach Anspruch 10, gekennzeichnet dadurch, daß der Puffer ein Acetat- oder ein Tris(hydroxymethyl)-aminomethanpuffer ist.

## Revendications

1. Une méthode permettant de détecter la présence d'une enzyme sélectionnée dans le groupe formé d'enzymes de type cellulase, lipase, ou amylase. Cette méthode est caractérisée par la production d'un substrat pour une enzyme donnée. Le substrat en question est modifié en étant marqué par un fluorophore et ensuite exposé à l'enzyme. Les réactions marquées de fluorescence produites sont finalement suivies.

2. Une méthode telle que citée dans l'énoncé 1, dans lequel les produits marqués de fluorescence de la réaction sont suivies au moyen d'un fluorimètre.

3. Une méthode telle que citée dans n'importe lequel des énoncés précédents, dans lequel l'enzyme est une enzyme de type cellulase et le substrat est de la cellulose.

4. Une méthode telle que citée dans n'importe lequel des énoncés précédents, dans lequel le fluorophore est jaune lucifer.

5. Une méthode telle que citée dans n'importe lequel des énoncés 1 à 3, dans lequel le fluorophore est de la fluorescéine ou un dérivé de fluorescéine.

6. Une méthode telle que citée dans n'importe lequel des énoncés précédents, dans lequel la méthode sera appliquée sur une base permanente en transportant l'air contenant l'enzyme de façon continue dans une solution et en passant ensuite la solution à base d'enzyme produite sur un lit imprégné du substrat marqué.

7. Une méthode telle que citée dans n'importe lequel des énoncés précédents, dans lequel le substrat marqué est transporté par un support.

8. Une méthode telle que citée dans l'énoncé 7, dans lequel le support est formé de billes de verre ou de particules de cellulose contenant de l'oxyde de fer.

9. Une méthode telle que citée dans l'énoncé 7, dans lequel le support est une fibre optique.

10. Une méthode telle que citée dans n'importe lequel des énoncés 6 à 9, dans lequel la solution contient un tampon.

11. Une méthode telle que citée dans l'énonce 10, dans lequel le tampon est un tampon acétate, ou un tampon tris-(hydroxymethyl)-aminomethane.
